# EUROPEAN PATENT APPLICATION

(11) **EP 2 633 832 A1**
(43) Date of publication of application: **04.09.2013**
(21) Application number: 11835678.1
(22) Date of filing: 28.10.2011
(51) Int. Cl.: A61B 19/02, A61L 11/00

(54) **CARTRIDGE FOR RECYCLING MEDICAL WASTE, WASTE CONTAINER AND METHOD FOR COMPACTING AND STERILISING SAID WASTE**

(30) Priority: 28.10.2010 WO PCT/ES2010/000458
(71) Applicant: Saraito, S.L., 18008 Granada (ES)
(72) Inventor: DUEÑAS SANCHEZ, Silverio, E-18008 Granada (ES)
(74) Representative: Manasse, Uwe
(86) International application number: PCT/ES2011/070751
(87) International publication number: WO 2012/056082

(57) **Abstract**

The invention relates to a cartridge (1) for recycling medical waste, which includes a body (2) of recyclable material with a closed shape, the inner structure of which is divided into multiple compartments that define chambers (3) closed by each one of the ends thereof, with axes that are parallel to one another and to the axis of the cartridge (1) and a heat-shrinkable plastic material (4) which covers the cartridge (1) at least on the sides. The invention relates to a waste container (5) which includes at least one waste-recycling cartridge (1). The invention also relates to a method for compacting and sterilising medical waste by using a recycling cartridge (1) and a waste container (5), wherein the phases that make up said method include inserting the recycling cartridge (1) in a container, filling the cartridge, inserting the two elements in a sterilisation autoclave (10), in which the cartridge (1) and the waste are sterilised, and shrinking the heat-shrinkable plastic material and compressing the recycling cartridge (1).

## Description

### Technical Field of the Invention

The present invention relates to the technical field of medical waste, specifically the collection thereof and subsequent treatments to which they are subjected.

### Background of the Invention

Currently there is a special sensivity for sanitary waste and collection and further processing of the same, due to the possible presence of germs in this waste, which badly treated may be the focus of diseases and infections.

In hospitals and medical campuses substantial waste is generated, many of them of a sharp and cutting in nature, such as syringes, vials, ... Special care must be taken that no one comes into contact with them as they have been previously used and discarded whereby, when not sterilised, they can transmit many infections.

There is also the risk that a person suffering from an injectable substance dependency, is faced with the temptation of accessing the container full of these products and try to take over any of them, without thinking about the multitude of possible additional risks.

That is why this type of waste requires special attention, both for the health personnel not to come into contact with them by mistake and such that there no possibility of extracting any of them on a voluntary basis.

To avoid this, there are currently a variety of containers and packaging models which try to collect this waste as carefully as possible to avoid contact therewith.

As examples of the prior art the following reference documents can be mentioned ES2296470, ES1024772-U, ES1018790-U, and ES1012620-U.

The first of these, patent ES2296470 discloses a stackable container with reduced dimensions, which at the top has an inlet port with a cap that can not be opened unintentionally.

The reduced dimensions allow lesser filling time and therefore reduce the residence time of each package in the collection site before bringing the product to its destruction.

In the case of the specification of reference Utility Model ES1024772-U, it is a container formed from a stamped sheet of cardboard that is folded longitudinally. This container stores the waste introduced therein by opening a cap, which has a top overcap to further prevent the exit of odors from inside the container.

In the case of the specification of reference Utility Model ES1018790-U, the waste collection container has a closure with a cap and a seal that generate a tight seal which does not allow the exit of waste in case of overturning the container and also does not allow the exit of odors or fumes from such waste.

Finally, in the specification of reference Utility Model ES1012620-U, there is disclosed a prismatic cardboard container with a deformable inner bag, of the dimensions of the container, in which waste is collected.

All of these references disclose waste collection systems that once removed are shipped to a waste treatment plant for destruction, not contemplating the option of sterilisation and subsequent recycling thereof.

If this sterilisation and recycling option would be raised, there would remain a same problem in all cases, because these containers are ready for destruction together with the waste, usually by incineration, but for sterilisation it would be necessary to extract the residues from the container, with the inherent risk that this entails.

Further, throughout the prior art there is no mention of the specific way in which such hazardous waste must be disposed of, in terms of transmission of infections, such as hypodermic syringes, being performed as if it was another residue.

This causes that when waste accumulates, it is pressed together, and the case may be that a needle traverses the container given the low resistance of its material, chosen in this way for easy destruction thereof.

As an extreme example of this problem mention could be made of the reference specification ES1012620-U, which discloses a deformable bag within the container, so that when it is full, the container is removed for destruction. In that process of extraction and transportation of waste, there are high possibilities that any needle or sharp object passes through the bag, with the risk that entails.

### Disclosure of Invention

The medical waste recycling cartridge herein disclosed comprises a body of recyclable material having a closed shape and an internal structure divided into multiple compartments.

These compartments define multiple chambers whose axes are parallel to each other and with the axis of the cartridge itself.

Said chambers are closed at one end and open at the opposite end, the latter being used for the introduction of waste therein.

It also comprises a heat-shrinkable plastic material covering the cartridge at least laterally.

The compartments into which the internal structure of the cartridge is divided have the particularity that the sectional dimensions are smaller than the length of the product to be deposited therein, so that the products are retained in each chamber in the longitudinal direction thereof. Thus, for example hypodermic syringes are all stored vertically along the axis of the cartridge.

The body of recyclable material forming the cartridge may be made of cardboard, and both this body and the heat-shrinkable plastic material overlying it preferably have a cylindrical shape.

This heat-shrinkable plastic material is preferably formed of polyethylene terephthalate (PET) or by a polylactic acid (PLA) derivative.

The cartridge may further comprise about the recyclable material forming the body thereof, an impregnation of a chemical product, preferably formed of a resin or powder chalk that when exposed to water or water vapor and cooling, undergoes a crystallization which generates its fast hardening, so that the cartridge itself is hardened.

Also, on the outer surface of the heat-shrinkable plastic material that covers the cartridge, it has a bar code for tracking of the cartridge as well as the existing materials inside.

Besides the recycling cartridge, a waste container is disclosed herein, which includes at least one recycling cartridge as defined above. The cartridge needs to be received within said container to be used comfortably and safely.

Thus, said container facilitates transportation of the cartridge from one room to another according to where it is needed at all times, avoiding any possible contact with the waste because it has a closing cap. This in turn has some hooks to separate needles from syringes.

Closing the cap can be by pressure, by clamping means or by clamping and screwing.

This waste container, which is preferably in a cylindrical shape, can be of metal or plastic.

Thus, its material is preferably stainless steel or plastic which cannot be deformed under the action of pressure steam autoclaving.

This container may comprise a series of holes on its lateral surface for the entry of water vapor in its interior, in the sterilisation process of the cartridge. In the case of closing the container by threaded means, they offer the option to open or close the openings by rotation of the screw of said cap. In this case it may be omitted placing the holes in the sides of the container body since positioning holes that can be pierced by the needle in normal use of the container is no longer permitted. With the placement of the holes in the threaded area such as in a normal way of use of the container, the holes are capped when the holes of the cap and the container do not coincide with each other and when the container is closed for sterilisation, the cap is tightened and the holes in the cap and the container are opposite, the water steam from the steriliser accessing into the container when it is sterilised.

Another option of the metallic container enabling the sterilisation process of the inner cartridge is one that does not include holes in its surface except for one for entry of the water vapor therethrough, which has a valve. In this case, said inlet valve comprises an antibacterial filter that prevents bacteria from exiting the container.

Besides the sanitary waste recycling cartridge, the compaction and sterilising procedure of medical waste by the use of a recycling cartridge and waste container as previously defined, are also disclosed herein.

This compaction and sterilising procedure of medical waste comprises first introducing the recycling cartridge in a waste container.

The cartridge is then filled with medical waste that the personnel deposits in its multiple longitudinal chambers.

Once the cartridge is filled, it is introduced together with the container inside an autoclave for sterilisation.

In this autoclave, due to water vapor, pressure and the high temperatures encountered therein, sterilisation both of the cartridge and the waste it contains is generated. At the same time, the high temperatures cause the shrinking of the heat-shrinkable plastic covering the cartridge, the waste being longitudinally compressed by the primary state of its location.

Shrinking of said plastic generates compression of the cartridge and the waste contained in its interior.

At this point, the container is removed from inside the autoclave, and finally the cartridge is removed from inside of said container for being transported to a recycling plant.

Having been compressed, the cartridge resulting from the process occupies a much smaller volume than it initially occupied.

With the recycling cartridge herein disclosed, significant improvements over the prior art are achieved.

Thus, on one hand the arrangement of storage chambers inside the same forces a longitudinal disposal of the waste, following the longitudinal axis of the cartridge itself. This is very important in the specific case of sharp and pinching waste such as hypodermic syringes because it avoids the possible breakage of the storage surface by a needle pressure just coming outside. This can occur when needles are stored in a free array so that by movement or the pressure itself against each other, one of them ends pinching the surface of the storage container, protruding to the outside, with the danger this entails.

With this way of filling the cartridge that does not happen, thus reducing the risk of infection or contagion by such discarded items.

Moreover, the existence of a heat-shrinkable plastic material covering the cartridge favors that when the cartridge is exposed to high temperatures, the volume thereof and its contents are reduced significantly, occupying much less space for storage until its transfer to a recycling plant. Also, by being all compressed, waste can not leave the cartridge neither accidentally nor voluntarily, which also adds security to the process.

Likewise, since the cartridge is made of recyclable material, in addition to reducing the cost thereof, it allows that when taken to a recycling plant the cartridge itself will be able to be recycled, along with the residues formed as itself by recyclable materials.

In turn, the waste container herein disclosed, also provides advantages over the prior art, since due to its mobility allows the cartridge existing therein to be readily transported to the room in which it is required and this in a totally safe way since it does not allow the removal of debris that already exist in the cartridge thanks to its cover, and also provides greater outer protection to the cartridge, since it creates an additional coating of the same.

Moreover, since the container is ready to withstand autoclave conditions, it is not necessary to remove the cartridge therein for introduction into the autoclave, because the container-cartridge assembly is introduced, thus avoiding any contact with the cartridge and therefore the risk that this would have.

Moreover, the compaction and sterilising procedure of medical waste herein proposed has also significant advantages over the prior art.

Thus, this procedure assumes a different option to what is currently being carried out consisting of a collection of waste for subsequent destruction usually by incineration in a waste treatment plant.

With the procedure herein disclosed, an alternative is provided that sanitary waste together with the cartridge itself that contains them, are first sterilised and compacted and then be transported to a recycling plant where each of the materials forming the assembly are separated.

They are sterilised to remove any viruses or bacteria they may contain, and compacted to reduce the space they occupy and also in order to convert both cartridge and waste into a whole one from which there can not be removed either accidentally or intentionally none of the waste it contains, because they are pressed by the heat-shrinkable sheath.

We see therefore that security is increased while reducing costs and contributing to protect the environment.

### Brief Description of the Drawings

In order to get a better understanding of the invention's characteristics, according to a preferred exemplary embodiment thereof, a set of drawings is provided as an integral part of said specification wherein, in an illustrative and non-limiting way, the following is shown:
Figure 1.- Shows a perspective view of the recycling cartridge.
Figure 2.- Shows a perspective view of a metal container of the type having a pressure and clamping cap closure.
Figure 3.- Shows a perspective view of the cartridge housed inside the metal container.
Figure 4.- Shows the introduction of a container with the cartridge in an autoclave.
Figure 5.- Shows a perspective view of the recycling cartridge after the compaction and sterilisation process, and once removed from the container for storage until recycling.
Figure 6 shows a detail of the exploited container cap in which the container holes are formed in the cap or the threaded end of the container.
Figures 7a and 7b show perspective views of the end of the container when the holes are open or closed, by simply the turning action of the threaded cap in order to match both holes formed in the container body and the cap.

### Detailed description of a preferred embodiment of the invention

In view of the figures provided, it can be seen how in one preferred embodiment of the invention, the cartridge (1) of sanitary waste recycling proposed here comprises a body (2) made of recyclable material that has a closed shape, being particularly in this preferred embodiment of the invention a cylindrical shape, like the material forming it is recyclable cardboard.

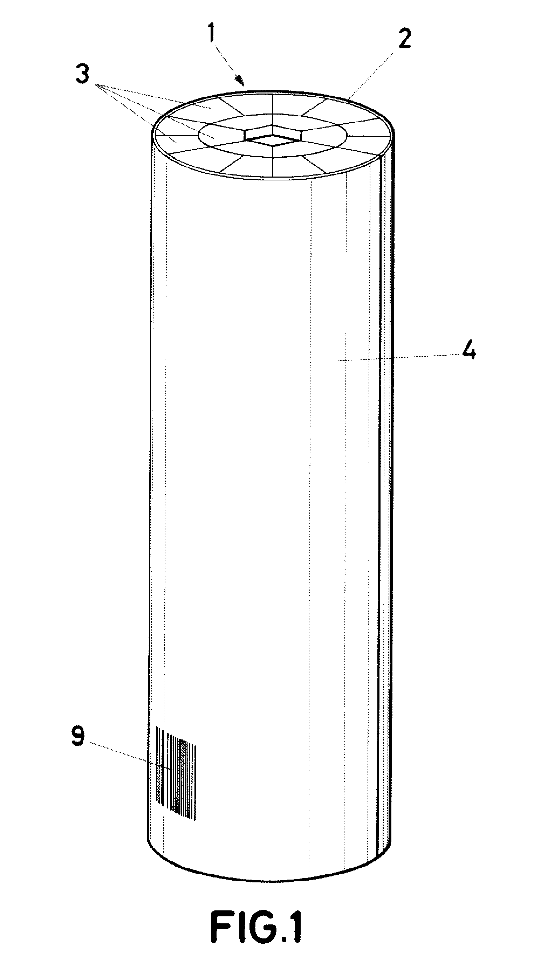

In Figure 1 it can be seen how the internal structure thereof is divided into multiple compartments which define multiple chambers (3) whose axes are parallel to each other and in turn to the axis of the cartridge (1).

These chambers (3) are closed at one end and open at the opposite end, through which filling of the cartridge (1) is carried out by introducing medical waste therein.

Likewise, the chambers (3) meet the condition that the sectional dimension of each is less than the length of the waste to be collected therein, so that the waste is stored in each chamber (3) longitudinally thereto.

In turn, this cartridge (1) has laterally a coating by a heat-shrinkable plastic material (4), which in this preferred embodiment of the invention has a cylindrical shape and is formed of polyethylene terephthalate (PET).

On the outer surface of the heat shrinkable plastic material (4), the cartridge (1) has a bar code (9) which promotes tracking of the cartridge (1) and the waste it contains.

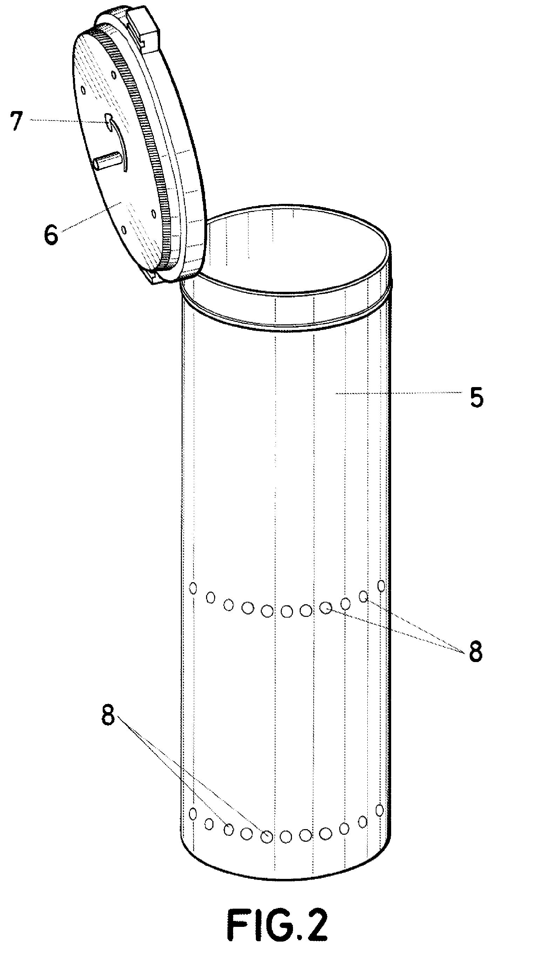

Furthermore, the recycling cartridge (1) is shown here with a container (5) of waste that can be seen in Figure 2 and that in this preferred embodiment of the invention has a cylindrical shape and is made of metal, being formed of stainless steel. Thus, the cartridge (1) for recycling is introduced therein thereby facilitating their use and transport without the possibility of contact with the waste contained therein.

In its cap (6), this container (5) has some hooks (7) for the separation of a needle from the syringe without contact therewith.

Likewise, the container (5) comprises in its lateral surface holes (8) for the passage of water vapor inside, necessary in a sterilisation process.

Apart from the cartridge (1) for recycling shown here, this specification discloses the process for compaction and sterilising of medical waste by the use of a cartridge (1) for recycling and a container (5) for waste as described above.

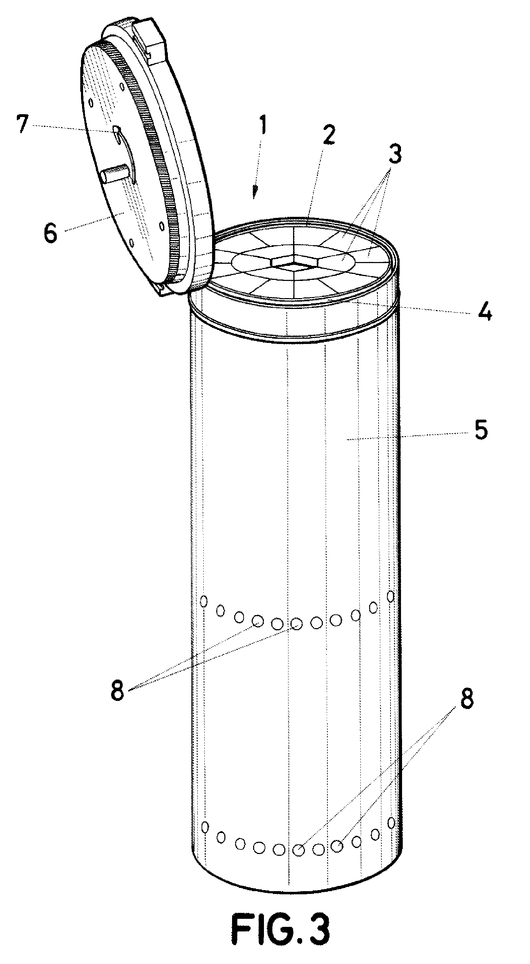

As seen in Figure 3 the first thing to do is to insert the cartridge (1) for recycling into the container (5). Once there and now without risk of contact with the waste, the cartridge (1) is filled with waste products that health personnel deposits therein and which longitudinal stay for the same.

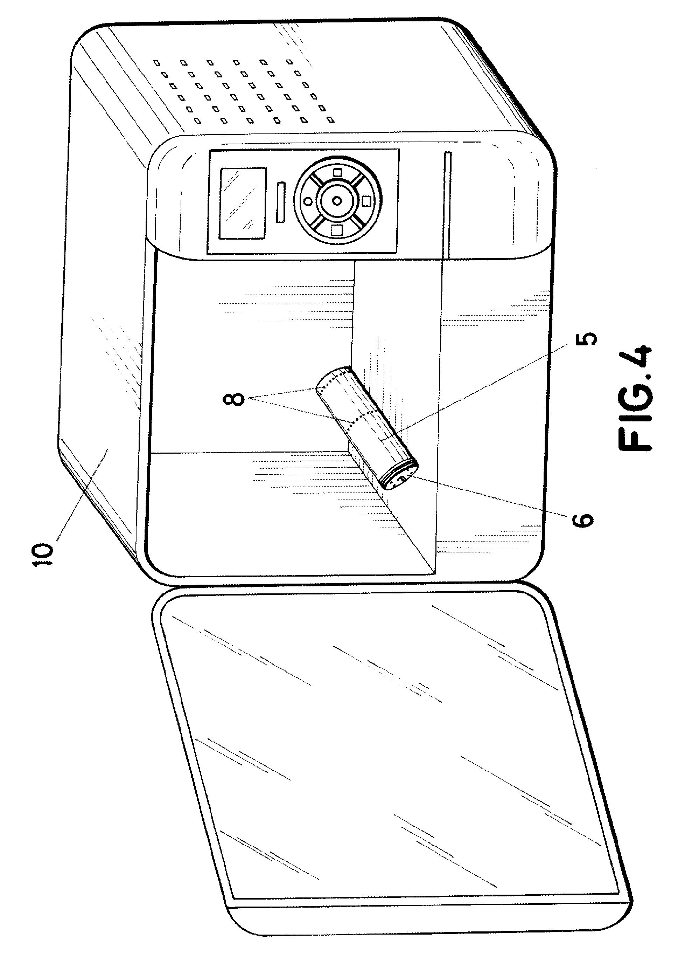

When the cartridge (1) is filled, it is deposited inside an autoclave (10) for sterilisation, as shown in Figure 4.

In this autoclave (10) for disinfecting, by steam and increased pressure and temperature, sterilisation of the cartridge (1) and the waste contained therein is performed.

The temperature rise experienced by the cartridge (1) causes the heat-shrinkable plastic (4) that covers it to retract, compressing the cartridge (1) and the waste therein.

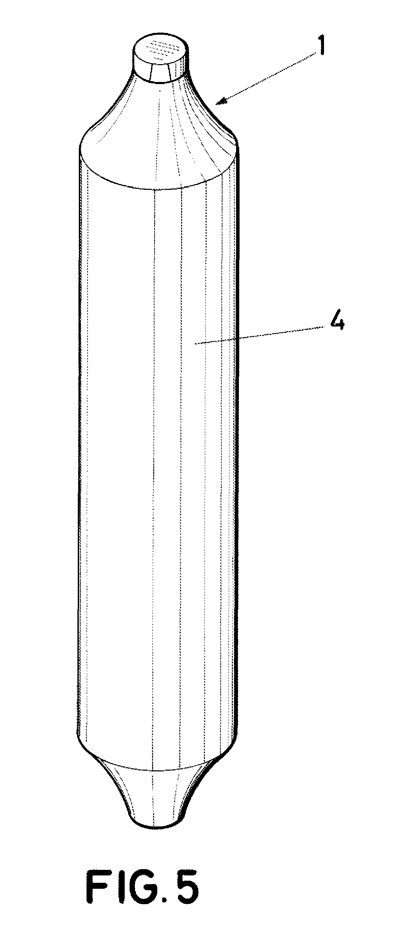

The container (5) with the cartridge (1) therein is removed from the autoclave (10) and subsequently this cartridge (1) for recycling is extracted from within the container (5). The result can be seen in Figure 5, which shows the cartridge (1) compressed due to retraction of the heat-shrinkable plastic (4) which surrounds it, so that the volume of the cartridge (1) with the waste therein is greatly reduced compared to what it originally occupied.

This cartridge (1) is stored until taken to a recycling plant.

With the recycling cartridge herein proposed, the state of the art is significantly improved because with a reduced cost and recyclable material as cardboard, a cartridge is manufactured for the collection and storage of medical waste which is easy to move from one room to another, depending on where it is needed.

The manner of storage to which this cartridge forces waste lengthwise the same is also an advantage over existing storage containers at present, because when forcing this position of waste the cutting elements are all controlled longitudinally and there is no possibility that a needle pierces the side surface of the cartridge, with the risk that this entails.

Likewise, the reduction in volume achieved when exposed to high temperature promotes storage thereof without problems.

The waste container also has advantages as it increases security assuming an additional coating of the waste cartridge during use.

It also facilitates a safe transport of the cartridge and in the sterilisation process of the cartridge offers the advantage of being able to withstand said procedure, not being necessary the removal of the cartridge, which always carries risks.

Regarding the procedure of compaction and sterilisation of medical waste herein disclosed, it also offers several advantages over the prior art mainly because it raises a different solution for medical waste, offering the alternative of sterilisation and subsequent recycling of products.

With this recycling, a diversity of materials are obtained that can be reused.

Moreover, by being sterilised and compacted at the same time the risk of possible contact with the waste is eliminated since, besides that when they are sterilised they no longer present the same risk, by being compacted, it is not possible to remove any residue present inside the cartridge with which it forms a compact assembly.

Therefore, costs are reduced, risk is minimized and the environment is respected, which is a very important issue today.

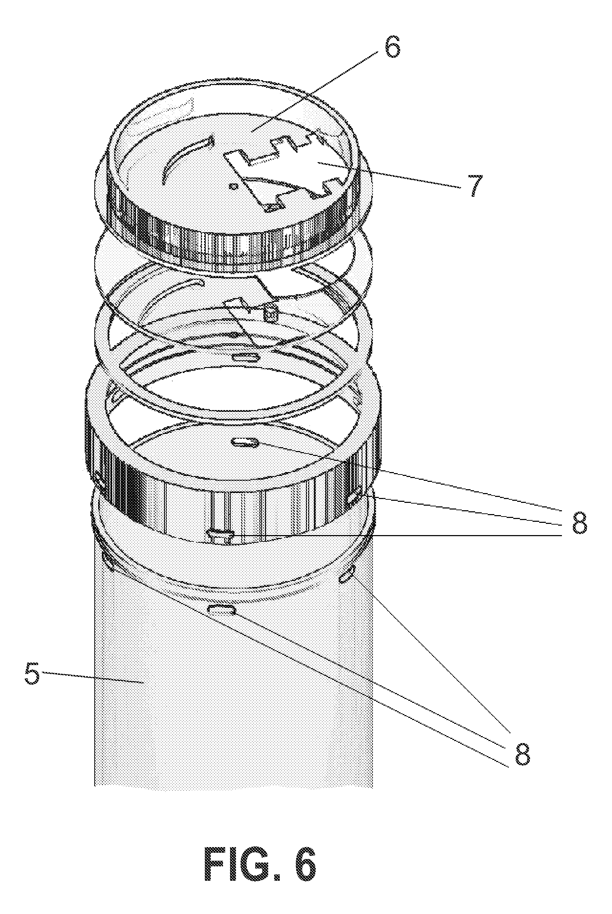

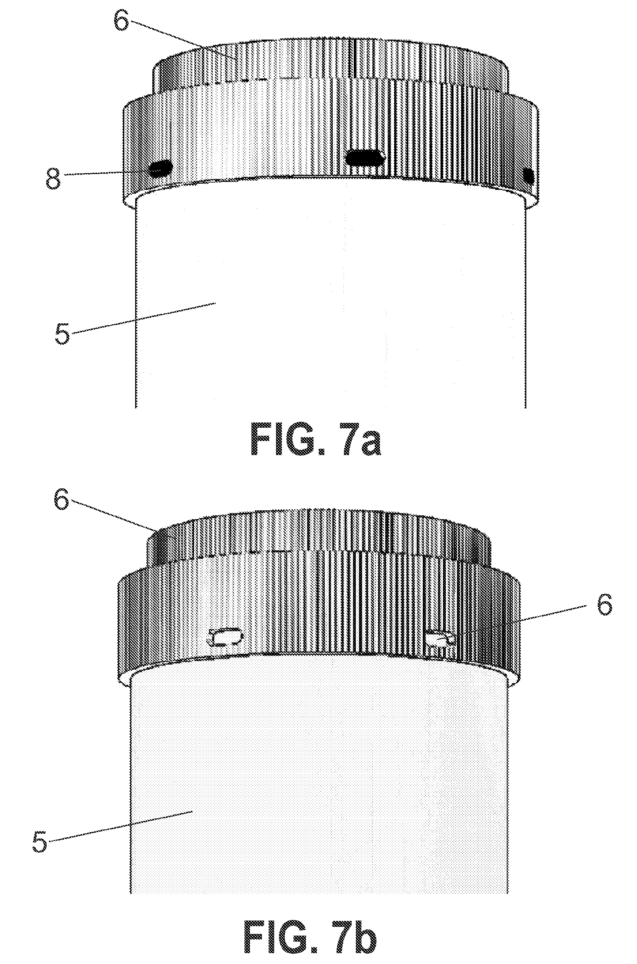

Figures 6, 7a and 7b show an advantageous solution of the container, which is a solution that lacks the opened lateral holes in the container, as shown in the previous figures. In this solution described in Figure 6 with an exploded view of its elements and in Figure 7a and 7b with respective representations of the open or closed holes, allows to see how the holes are arranged both on the cap and in the threaded neck of the container, in this case, a rotation to a predetermined position the container would be in position to be filled with waste products and the side holes are closed because those incorporated in the cap and in the threaded neck of the container are not brought into coincidence.

Once the entire container is filled and before sending to the steriliser, the cap is finally threaded, at which time the holes of the cap and the container are placed in communication, the inside of the container being connected to the outside through desired holes, being able to enter the vapor from the steriliser when subjected to this process.

## Claims

1. Cartridge (1) for medical waste recycling, **characterized by** comprising
- a body (2) of recyclable material that is configured in a closed shape, whose internal structure is divided into multiple compartments which define multiple chambers (3) whose axes are parallel to each other and parallel to the axis of the cartridge (1), these chambers (3) being closed at one end and open at the opposite end for inserting the waste material therein; and
- a heat-shrinkable plastic material (4) covering the cartridge (1) at least laterally.

2. Cartridge (1) for recycling according to claim 1, **characterized in that** the sectional dimensions of the compartments which form the internal structure thereof, are less than the length of the product to be deposited therein, said products being retained in a longitudinally direction to the chamber (3) where it is housed.

3. Cartridge (1) for recycling of any of the preceding claims, **characterized in that** the body (2) of recyclable material has a cylindrical shape.

4. Cartridge (1) for recycling of any of the preceding claims, **characterized in that** the heat-shrinkable plastic material (4) adopts a cylindrical section.

5. Cartridge (1) for recycling of any of the preceding claims, **characterized in that** the body (2) of recyclable is preferably formed of cardboard.

6. Cartridge (1) for recycling of any of the preceding claims, **characterized in that** the heat-shrinkable plastic material (4) covering it is preferably formed of polyethylene terephthalate (PET) or a polylactic acid (PLA) derivative.

7. Cartridge (1) for recycling of any of the preceding claims, **characterized in that** the recyclable material forming it is impregnated with a chemical product, preferably a resin or gypsum powder that when being exposed to water vapor and cooling, undergoes crystallization and hardens quickly.

8. Cartridge (1) for recycling of any of the preceding claims, **characterized by** comprising on the outer surface of the heat-shrinkable plastic material (4), a bar code (9) for the traceability of the cartridge (1) and the materials contained therein.

9. Container (5) of waste that includes at least one cartridge (1) for recycling waste as defined in claims 1 to 8, **characterized in that** it can be of metal or plastic.

10. Container (5) of waste according to claim 9, **characterized in that** it is preferably formed of stainless steel or plastic.

11. Container (5) of waste of any of the preceding claims, **characterized in that** it has a cylindrical shape.

12. Container (5) of waste of any of the preceding claims, **characterized in that** the closure cap comprises closure means preferably by screwing or fastening tightening.

13. Container (5) of waste according to any preceding claim, **characterized by** comprising a series of holes (8) on its lateral surface for entrance of water vapor.

14. Container (5) of waste according to any one of claims 9 to 11, **characterized by** having a side surface without holes comprising a single inlet for the water vapor through a valve.

15. Container (5) of waste according to any one of claims 1 to 13, **characterized in that** the holes (8) are arranged in the threading area of the container cap, opening or closing the passage of air and steam inside the container by rotating the screw cap on the container end.

16. Container (5) of waste according to claim 14, **characterized in that** the inlet valve to the container (5) comprises an antibacterial filter that prevents exit thereof.

17. Procedure for compaction and sterilising medical waste by the use of a cartridge (1) for recycling and a container (5) of waste as defined in claims 1 to 16, **characterized by** comprising
- introducing the cartridge (1) for recycling in a container (5)
- filling the chambers (3) of the cartridge (1) with waste
- introducing the cartridge (1) for recycling together with the container (5) in which is housed, inside an autoclave (10) for sterilisation,
- sterilising the cartridge (1) and the waste contained therein and shrinkage of the heat-shrinkable plastic material (4) that covers the cartridge (1) due to high temperature, pressure and water vapor in the disinfection autoclave (10),
- compressing the cartridge (1) for recycling carrying the waste materials, due to the retraction of the heat-shrinkable plastic material (4),
- removing the container (5) from inside the autoclave (10); and
- removing the cartridge (1) with the shrunken plastic for recycling materials forming the same.
